# EUROPÄISCHE PATENTANMELDUNG

(11) **EP 0 702 024 A1**
(43) Veröffentlichungstag der Anmeldung: **20.03.1996**
(21) Anmeldenummer: 95110920.6
(22) Anmeldetag: 12.07.1995
(51) Int. Cl.: C07H 15/04

(54) **Verfahren zur Herstellung von Alkylpolyglycosiden**

(30) Priorität: 07.09.1994 DE 4431856
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Die Erfindung betrifft ein neues Verfahren zur Herstellung von Alkylpolyglycosiden aus Alkylglycosiden mit C₁- bis C₆-Alkylresten und Alkoholen mit 8 bis 20 C-Atomen durch säurekatalysierte Umglycosidierung. Dabei wird die Reaktion mit Hilfe eines Rohrreaktors unter Gleichstrombedingungen durchgeführt.

## Beschreibung

Die Erfindung betrifft ein neues Verfahren zur Herstellung von Alkylpolyglycosiden mit C₈- bis C₂₀-Alkylresten aus Alkylglycosiden mit C₁- bis C₆-Alkylresten und Alkoholen mit 8 bis 20 C-Atomen durch säurekatalysierte Umglycosidierung.

Alkylpolyglycoside mit C₈- bis C₂₀-Alkylresten können ganz oder teilweise aus nachwachsenden Rohstoffen hergestellt werden. Die Alkylpolyglycoside gewinnen wegen ihrer interessanten Tensideigenschaften bei gleichzeitig sehr guter biologischer Abbaubarkeit zunehmend an Bedeutung. Für Anwendungen im Haushalt und im Kosmetikbereich müssen diese Produkte hohen ästhetischen Ansprüchen genügen. Man ist daher an Verfahren interessiert, nach denen Alkylpolyglycoside in transparenten, farblich schönen wäßrigen Lösungen hergestellt werden können.

Zur Herstellung von Alkylpolyglycosiden mit langkettigen Alkylgruppen kann man zunächst durch Glycosidierung von Sacchariden mit kurzkettigen Alkoholen Alkylglycoside mit C₁- bis C₆-Alkylgruppen herstellen. Diese Produkte werden dann mit langkettigen Alkoholen durch Umglycosidierung bei erhöhter Temperatur in die gewünschten Alkylpolyglycoside übergeführt. Die so hergestellten Produkte sind jedoch dunkel gefärbt.

Unter Einhaltung bestimmter Mengenverhältnisse und bei Verwendung von Löslichkeitsvermittlern können die polaren Saccharide auch direkt mit den unpolaren langkettigen Alkoholen zu den Alkylpolyglycosiden umgesetzt werden. Dabei werden auch hier ohne Zusatz von farbverbessernden Mitteln, wenn die Reaktion im Rührkessel durchgeführt wird, dunkelfarbige Produkte erhalten.

In EP 0 077 167 wird ein einstufiges Herstellverfahren beschrieben, bei dem eine Aldose oder eine Ketose direkt mit einem langkettigen Alkohol im Molverhältnis von 1 : 1,25 bis 1 : 4 umgesetzt wird. Die Reaktion wird bei geringen Wassergehalten in Gegenwart eines Reduktionsmittels durchgeführt.

Dieses einstufige Verfahren wird in DE-A-41 01 252 dadurch verbessert, daß man einen großen Alkoholüberschuß verwendet und zur Neutralisation in Alkoholen gelöstes Alkalihydroxid verwendet. Die Reaktion wird hier in einem Rührkessel ausgeführt.

Darüber hinaus ist bekannt, daß man die Farbqualität der Alkylpolyglycoside durch apparative Maßnahmen verbessern kann.

So kann man nach EP-A-0 482 325 bei einem zweistufigen Herstellverfahren die 1. Stufe, die Glycosidierung von Sacchariden in wäßriger Lösung mit C₁- bis C₆-Alkoholen, in einer Gegenstromreaktionskolonne, beispielsweise in einer Glockenbodenkolonne, durchführen.

Außerdem kann nach DE-A-41 16 665 auch die Umglycosidierung, die 2. Stufe des zweistufigen Verfahrens, in einer Reaktionskolonne und vorzugsweise unter Gegenstrombedingungen ausgeführt werden.

Die genannten Verfahren können hinsichtlich der Reaktoren noch weiter verbessert und vereinfacht werden.

Aufgabe der vorliegenden Erfindung war es daher, in einem zweistufigen Verfahren die Umglycosidierung weiter zu vereinfachen.

Die Aufgabe wird erfindungsgemäß dadurch gelöst, daß man die Umglycosidierung mit Hilfe eines Rohrreaktors unter Gleichstrombedingungen durchführt.

Geeignete Rohrreaktoren haben im allgemeinen einen Durchmesser von 0,5 bis 50 cm und eine Länge von 0,5 bis 50 m, wobei die Länge mindestens das 4fache des Durchmessers sein soll. Im einfachsten Falle handelt es sich dabei um ein beheizbares Rohr. Vorzugsweise weist der Rohrreaktor jedoch auch mehrere Stutzen auf, über die leicht flüchtige Stoffe mittels Vakuum oder mit Hilfe eines Inertgasstroms abgezogen bzw. ausgetrieben werden können.

Der Rohrreaktor kann auch Schikanen und Verwirbelungseinbauten enthalten. Das Rohr kann horizontal, schräg oder vertikal angeordnet sein. Kolonnen werden jedoch im Sinne dieser Erfindung nicht als Rohrreaktoren angesehen.

Der Saccharidteil der eingesetzten Alkylglycoside kann von Aldosen oder Ketosen hergeleitet werden. Beispiele dafür sind Glucose, Mannose, Galaktose und Fructose. Dabei wird Glucose vorzugsweise verwendet. Als Alkylreste kommen beispielsweise Methyl, Ethyl, Butyl oder Hexyl in Betracht.

Für das vorliegende Verfahren geeignete Alkohole sind beispielsweise Octanol, Decanol, Laurylalkohol, Myristyl-, Palmityl- und Stearylalkohol. Es können auch Gemische von Alkoholen eingesetzt werden. Vorzugsweise verwendet man Alkohole mit 8 bis 12 C-Atomen.

Das Alkylglycosid/Alkohol-Molverhältnis liegt bevorzugt im Bereich von 1 : 2 bis 1 : 10.

Als Katalysatoren sind Mineralsäuren und starke organische Säure geeignet. Beispiele dafür sind Schwefelsäure, Phosphorsäure und p-Toluolsulfonsäure. Der Katalysator wird vorzugsweise in Konzentrationen von 0,2 bis 5 %, bezogen auf das Saccharid, eingesetzt.

Die Reaktion wird meist bei einer Temperatur von 70 bis 140 °C durchgeführt. Dabei werden Temperaturen von 100 bis 140 °C besonders bevorzugt.

Gleichzeitig wird vorzugsweise eine mittlere Verweilzeit von 5 bis 90 Minuten eingestellt.

Das Verfahren wird vorzugsweise kontinuierlich durchgeführt. Die Produkte weisen im allgemeinen einen mittleren Glycosidierungsgrad von 1 bis 10 auf, wobei mittlere Glycosidierungsgrade von 1,1 bis 4 besonders bevorzugt eingestellt werden. Ganz besonders bevorzugt werden mittlere Glycosidierungsgrade von 1,1 bis 1,5.

Durch die vorliegende Erfindung wird insbesondere bei kontinuierlich arbeitenden Syntheseanlagen der apparative Aufwand stark vermindert. Der Umsatz wird gesteigert, die Raum-Zeit-Ausbeute verbessert. Durch die kurzen Verweilzeiten und die engen Verweilzeitverteilungen erhält man wegen der geringen thermischen Belastung helle Produkte von hoher Qualität. Hellfarbig sind die Produkt, wenn ihre 50%igen wäßrigen Lösungen vor der H₂O₂-Bleichung Jodfarbzahlen von < 60 aufweisen.

Nach der Reaktion wird das Produkt in bekannter Art und Weise mit einer Base neutralisiert, worauf überschüssiger Fettalkohol destillativ abgetrennt wird. Der Restfettalkoholgehalt liegt dann im allgemeinen bei unter 1 %. Anschließend wird das Produkt im allgemeinen mit Wasser abgemischt und mit H₂O₂ gebleicht.

Bei der praktischen Durchführung der Erfindung können dem Rohrreaktor auch Rührkessel vor- oder nachgeschaltet sein. So können die Alkylglycoside mit den langkettigen Alkoholen beispielsweise in einem vorgeschalteten Rührreaktor zu einem Teil reagieren. Das Reaktionsgemisch wird dann homogenisiert und in den Rohrreaktor geleitet. Nachgeschaltete Rührreaktoren dienen im allgemeinen nur dazu, die Verweilzeit zu erhöhen und den Umsatz zu vervollständigen. Vorzugsweise werden 5 bis 80 % der Reaktion in einem Rohrreaktor durchgeführt.

### Beispiel

Ein Rührreaktor wird kontinuierlich mit 40 kg/h Fettalkoholgemisch aus 68 % Dodecanol, 26 % Tetradecanol und 6 % Hexadecanol sowie mit 20 kg/h butanolischer Butylglucosidlösung, die 65 % Butanol und 35 % Butylglucosid enthält, beschickt. Dabei wird eine mittlere Verweilzeit von 3,5 Stunden eingestellt. Durch Zudosieren von p-Toluolsulfonsäure wird eine Katalysatorkonzentration von 0,25 Gewichtsprozent im Reaktionsgemisch aufrechterhalten. Bei 115 °C und 5 mbar weren über eine Kolonne ca. 13 kg/h Butanol abdestilliert. Den Rührreaktor verlassen stündlich 47 kg Reaktionsgemisch, das 77 % Fettalkohol, 11,1 % C₁₂- bis C₁₆-Alkylglucosid und 0,63 % Butylglucosid enthält.

Dieses Reaktionsgemisch wird bei 112 °C durch ein Rohr mit einem Innendurchmesser von 25 cm und einer Länge von 200 cm geleitet. Bei einer theoretischen Verweilzeit von ca. 30 Minuten erhält man am Ende des Rohrreaktors ein Gemisch, das 76,5 % Fettalkohol, 11,7 % C₁₂- bis C₁₆-Alkylglucosid und 0,26 % Butylglucosid aufweist. Der mittlere Glycosidierungsgrad des Alkylglucosids liegt bei 1,2.

## Patentansprüche

1. Verfahren zur Herstellung von Alkylpolyglycosiden mit C₈- bis C₂₀-Alkylresten aus Alkylglycosiden mit C₁- bis C₆-Alkylresten und Alkoholen mit 8 bis 20 C-Atomen durch säurekatalysierte Umglycosidierung,
dadurch gekennzeichnet,
daß man die Reaktion mit Hilfe eines Rohrreaktors unter Gleichstrombedingungen durchführt.

2. Verfahren nach Anspruch 1,
dadurch gekennzeichnet,
daß man die Reaktion bei 100 bis 140 °C durchführt.

3. Verfahren nach Anspruch 1,
dadurch gekennzeichnet,
daß man bei der Reaktion eine mittlere Verweilzeit von 5 bis 90 Minuten einstellt.

4. Verfahren nach Anspruch 1,
dadurch gekennzeichnet,
daß man Alkohole mit 8 bis 12 C-Atomen umsetzt.

5. Verfahren nach Anspruch 1,
dadurch gekennzeichnet,
daß man 5 bis 80 % der Reaktion in einem Rohrreaktor durchführt.
